# EUROPEAN PATENT SPECIFICATION

(11) **EP 2 612 602 B1**
(45) Date of publication and mention of the grant of the patent: **26.06.2019**
(21) Application number: 11821437.8
(22) Date of filing: 15.07.2011
(51) Int. Cl.: A61B 10/00

(54) **TOUCH PERCEPTION SENSITISING GLOVE**
HANDSCHUH ZUR SENSIBILISIERUNG FÜR BERÜHRUNGSREIZE
GANT SENSIBILISANT AUX PERCEPTIONS TACTILES

(30) Priority: 01.09.2010 JP 2010195328
(43) Date of publication of application: 10.07.2013
(60) Divisional application: 19166126.3
(73) Proprietor: ICST Corporation, Saitama-shi, Saitama 338-0001 (JP)
(72) Inventor: YOKOI, Hiroyuki, Saitama-shi Saitama 338-0001 (JP)
(74) Representative: TBK
(86) International application number: PCT/JP2011/066191
(87) International publication number: WO 2012/029419

(56) References cited:
- JP-A- 61 292 182
- JP-A- 2005 185 448
- JP-A- 2007 325 606
- JP-U- H03 102 495
- US-A1- 2003 195 437
- US-A1- 2006 090 243
- US-A1- 2007 271 676
- US-A1- 2008 173 299
- US-B2- 6 506 170

## Description

### Field

The present invention relates to a glove for sensitizing the sense of a hand when performing palpation for medical purposes, checking a surface finishing state of an industrial product, or the like.

### Background

Palpation, i.e., an examination for the condition of the surface or inside of a patient's body by means of the touch perception of a hand, has been conventionally performed in medical practice or the like. Evaluating a body condition accurately with a hand leads to an early detection for the cause of disease. Moreover, since a patient can perform palpation by oneself too, it is meaningful also in a situation of a self-health check or the like. In a breast cancer screening, for example, the condition of the chest is typically checked with a hand. An incidence of breast cancer has been increasing especially in recent years, and the early detection thereof has been therefore a very important issue. Breast cancer forms a "lump" when developed in the chest. Thus, if breast cancer can be detected with palpation while the lump is still small, the mortality rate can be reduced by providing early treatment.

In general, palpation is performed by moving a hand across the chest when the skin is wet or the skin is soaped over while taking a bath or the like so as to check if there is a lump formed inside the chest. This is because the condition inside the body can be felt acutely by reducing the frictional resistance between the hand and the chest. In an environment other than bathing, on the other hand, since a frictional resistance is generated between the hand and the chest, the sensitivity of the hand is reduced. As a result, there may be a case where a lump cannot be detected accurately.

In view of this, there has been known a pad with which the touch perception of a hand is sensitized regardless of the skin condition of the body so that a lump inside the body can be detected at an early stage (see Patent Literature 1, for example). This pad has a configuration such that a lubricating liquid such as silicon or glycol is retained inside a covering (bag) made of a latex material or a natural rubber material. By moving a hand while holding this pad between the chest and the hand, friction is reduced due to the action of the lubricating liquid. It is made to palpate the chest via the lubricating liquid and the covering made of a latex material or the like.

There is also a bag-shaped examination glove to be used with a hand being inserted therein (see Patent Literature 2, for example) . In this examination glove, a contact-side fabric to be brought into contact with the chest has a two-layer structure. By making the two-layered fabrics slide relative to each other, the touch perception of a hand is sensitized.

Patent Literature 3 discloses a glove for sensitizing touch perception having the features of the preamble of claim 1. Further prior art is discussed in Patent Literature 4, 5 and 6.

### Citation List

### Patent Literature

Patent Literature 1: Japanese Patent Application Laid-Open No. Sho. 61-292182
Patent Literature 2: US Patent Application Publication No. 2002/0169389
Patent Literature 3: US 2007/271 676 A1
Patent Literature 4: US 2006/090 243 A1
Patent Literature 5: US 2003/195 437 A1
Patent Literature 6: US 2008/173 299 A1

### Summary

### Technical Problem

However, the above-described pad had problems that when the covering thereof is ruptured due to some sort of outer shock, the lubricating liquid inside is leaked into outside and the pad therefore becomes unusable and the liquid also dirties the surrounding area thereof. Moreover, each material needed to be selected so as to prevent the lubricating liquid from seeping through the covering. If the selection was mistaken, it had a problem that the lubricating liquid moistens the covering and gradually seeps therethrough.

Moreover, although the combination of the lubricating liquid of a silicon material and the covering of a latex material, or the like, has a property that they are less likely to moisten each other, there has been a problem that the raw materials themselves are expensive. Thus, in spite of the extremely-high needs for the pad itself, the pad has not come into wide common use yet.

Further, if this pad is used in a standing position, the lubricating liquid therein is accumulated in a lower part inside the covering due to the gravity. Thus, the lubricating liquid lacks in an area from the middle to the upper part of the pad, thereby leading to a problem that palpation is difficult to be performed.

The above-described examination glove, on the other hand, employs no lubricating liquid and has a configuration fixable to a hand. As a result, the above-described examination glove is more user-friendly as compared to the above-described pad. In the above-described examination glove, however, an inner sheet having a size larger than that of an outer sheet is sewn to the outer sheet with tucks. As a result, wrinkles or rucks are more likely to be generated on the inner sheet, leading to problems that it influences subtle touch perception of a fingertip and the inner sheet is difficult to be slid smoothly relative to the outer sheet.

Moreover, it is made to insert a hand deep inside the glove and to be used with fingertips being positioned in the vicinity of seams at the tip of the glove. As a result, there has been a problem that the relative movement between the inner sheet and the outer sheet is blocked by the seams. Specifically, when performing palpation while sliding the inner sheet relative to the outer sheet with fingertips, the movement of the fingertips is limited by the immediate seams. Thus, there has been a problem that it is more likely to result in an inadequate examination.

The present invention has been made in view of the above-described problems, and an object thereof is to provide a glove for sensitizing touch perception with which fingertips can be moved smoothly across a wide area without influencing subtle touch perception and an examination by means of palpation can be performed highly accurately and smoothly.

### Solution to Problem

The object is achieved by a glove for sensitizing touch perception having the features of independent claim 1. Further advantageous developments are set out in the dependent claims.

### Advantageous Effects of Invention

According to the glove for sensitizing touch perception of the present invention, since fingertips can be moved smoothly across a wide area without influencing subtle touch perception, there can be provided an excellent advantageous effect such that an examination by means of palpation can be performed highly accurately and smoothly.

### Brief Description of Drawings

FIG. 1(a) is a plan view of a glove for sensitizing touch perception according to an embodiment of the present invention. FIG. 1(b) is a cross-sectional view taken along a line A-A in FIG. 1(a).
FIGs. 2(a) and 2(b) each are a cross-sectional view showing a state at the beginning of the use of the glove.
FIG. 3 is a plan view showing a state in which a hand is being inserted into the glove.
FIGs. 4(a) to 4(c) each are a cross-sectional view schematically showing a state during the use of the glove in a breast cancer screening.
FIGs. 5(a) and 5(b) each are a plan view showing a not claimed comparative example in which stoppers are configured so as to come into contact with portions between fingers of a hand inserted between a sensitizing layer and an external cover.
FIGs. 6(a) and 6(b) each are a plan view showing an example in which the stopper is configured so as to come into contact with a portion between the thumb and the index finger.
FIGs. 7(a) and 7(b) each are a plan view showing an example for another shape of a partially-connected portion forming the stopper to come into contact with a portion between the thumb and the index finger.
FIGs. 8(a) and 8(b) each are a plan view showing a not claimed comparative example in which the stopper is configured by closing part of an insertion opening.
FIG. 9 is a plan view showing a not claimed comparative example in which the insertion opening is formed to have a width allowing a hand to be inserted partially.
FIGs. 10(a) to 10(b) each are a plan view, wherein FIG. 10(a) shows an example and FIG. 10(b) shows a not claimed comparative example, in which a size of the external cover in a planar view is made smaller than that of the sensitizing layer.
FIGs. 11(a) and 11(b) each are a view showing a not claimed comparative example in which a tip side (fingertip side) of the external cover is opened.

### Description of Embodiments

Exemplary embodiments of the present invention and not claimed comparative examples useful for understanding the invention will now be described below in detail with reference to the accompanying drawings.

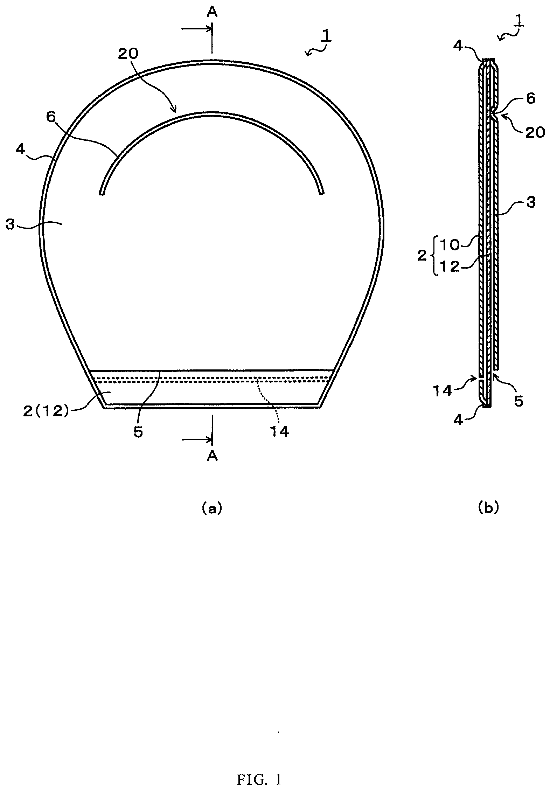

FIG. 1(a) shows a plan view of a glove for sensitizing touch perception (hereinafter referred to as glove) 1 according to the present embodiment. FIG. 1(b) shows a cross-sectional view taken along a line A-A in FIG. 1(a). Note that a size in a thickness direction is shown in a stretched manner in each of cross-sectional views of the glove 1 in FIG. 1(b) and the following drawings in order to facilitate understanding.

As shown in FIGs. 1(a) and 1(b), the glove 1 is configured to have a planar view shape obtained by cutting off part of an oval shape linearly. The glove 1 includes a sensitizing layer 2 to be in contact with an object and an external cover 3 for fixing a hand to the sensitizing layer 2.

The sensitizing layer 2 has a first film 10 to be in contact with an object and a second film 12 layered on the first film 10. The first film 10 and the second film 12 are adhered to each other at a connected portion 4 at a peripheral edge thereof, and are relatively movable in a portion on the inner side of the connected portion 4.

The sensitizing layer 2 is provided with an air introduction opening 14. By introducing air between the first film 10 and the second film 12, they are prevented from adhering closely to each other. As a result, the second film 12 can smoothly move relative to first film 10. In the present embodiment, the air introduction opening 14 is formed in a slit shape in the first film 10 in the vicinity of the inner side of a straight portion of the peripheral edge. Moreover, by forming the air introduction opening 14 on the side of the first film 10, the air introduction opening 14 is opened to the side of an object.

The external cover 3 is layered on the second film 12 of the sensitizing layer 2. In a similar manner to the first film 10 and the second film 12, the external cover 3 is adhered to the second film 12 at the connected portion 4 at the peripheral edge thereof. Note however that a straight portion of the peripheral edge of the external cover 3 is not adhered to the second film 12 and this portion serves as an insertion opening 5 to be used for inserting a hand between the sensitizing layer 2 (the second film 12 thereof) and the external cover 3. In other words, the sensitizing layer 2 and the external cover 3 are connected to each other at the respective peripheral edges thereof to form a bag shape and a hand is inserted between the sensitizing layer 2 (the second film 12 thereof) and the external cover 3 through the insertion opening 5. In the present embodiment, the insertion opening 5 is located on the slightly inner side of the peripheral edge straight portion of the sensitizing layer 2. As a result, the position of the insertion opening 5 can be easily recognized and a hand can be easily inserted therein.

Although the illustration is omitted in the figures, the external cover 3 is provided with a plurality of air holes for releasing moisture from a hand to the outside. As a result, even during a use for a prolonged time, dampness is prevented and a comfortable use is therefore possible.

The external cover 3 and the second film 12 of the sensitizing layer 2 are connected (adhered) to each other at a partially-connected portion 6 provided in a portion on the slightly inner side of the peripheral edge thereof. The partially-connected portion 6 forms a stopper 20 for positioning a hand inserted between the second film 12 and the external cover 3. In the present embodiment, the stopper 20 is provided in this manner so as to prevent a deep insertion between the second film 12 and the external cover 3 and to allow fingertips for performing palpation to be located at a position enabling the smooth movement thereof together with the second film 12. The detailed description of the function of the stopper 20 will be described later.

Transparent materials are used as materials for forming the sensitizing layer 2 and the external cover 3. In the present embodiment, a colorless and transparent material is used for the sensitizing layer 2 (i.e., the first film 10 and the second film 12), and a pink-colored and transparent material is used for the external cover 3. By employing the colors (including the transparent color) different from each other for the sensitizing layer 2 and the external cover 3 in this manner, the insertion opening 5 into which a user inserts a hand can be easily found visually. Note that the insertion opening 5 may be indicated clearly not by coloring the materials themselves but by partially printing colors, patterns, figures, signs, or the like on each of the films.

A synthetic resin material is used for the first film 10 and the second film 12. Particularly in the present embodiment, an ethylene-vinyl acetate copolymer (EVA) film with a thickness of 0.10 mm is used. A thickness of the each film is preferably 0.10 mm or thinner, and more preferably 0.03 mm or thinner. The ethylene-vinyl acetate copolymer has a low price but realizes a favorable sliding between the films. The softness thereof is also suitable for sensitizing touch perception. Moreover, unlike a chlorine-based material, it also has an advantage that a harmful substance is less likely to be generated upon the disposal thereof.

Other than the ethylene-vinyl acetate copolymer, a synthetic resin material such as a polyolefin such as polyethylene (PE) or polypropylene (PP), a polyester such as polystyrene (PS), polyethylene terephthalate (PET), or polyethylene isophthalate (PEI), or a homopolymer or a copolymer such as nylon (NY), polyvinyl chloride (PVC), an ethylene vinyl alcohol copolymer (EVOH), polyvinyl alcohol (PVA), or polyvinylidene chloride (PVDC) can also be used, for example. Among these, polyethylene or ethylene vinyl alcohol is a preferable material.

For the first film 10 and the second film 12, these synthetic resins may be used independently, or a film formed by combining these synthetic resins or a layered film obtained by layering these synthetic resins may be used. Further, a known coating agent may be coated on the surfaces of these synthetic resin films in order to impart thereto an easy sliding property, an antistatic property, and the like.

Note that in the present embodiment, a durometer indentation D hardness of the first film 10 and the second film 12 is set to be smaller than or equal to 36 (SHORE D) . As a result, each film can be easily bent when the first film 10 and the second film 12 are slid relative to each other. Thus, it is possible to prevent the rigidity of the material itself from acting as a resistance. Note that the durometer indentation Dhardness (SHORE Dhardness) is defined in ISO7619, ISO868, and the like, and is a value measured by pressing a measuring needle into a material.

An elongation percentage of the first film 10 and the second film 12 is preferably set to be 700% or more. As a result, the films are easily elongated and contracted during palpation and can therefore follow the elongation and contraction of the skin. Further, a rupture strength in a case where the film thickness is 0.03 mm is preferably set to be greater than or equal to 2.7 N. Although the material identical with that for the first film 10 and the second film 12 is used also for the external cover 3 in the present embodiment for the ease of the manufacturing process thereof, materials different from each other may be used instead.

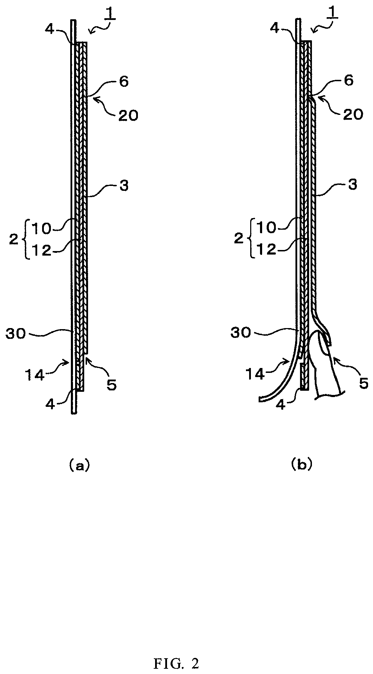

FIGs. 2(a) and 2(b) each are a cross-sectional view showing a state at the beginning of the use of the glove 1. Here, FIG. 2(a) shows a packaged state when the glove 1 is provided to a user as a product. A base sheet 30 made of a paper is temporarily adhered to the first film 10 side of the glove 1 in a packaged state. The base sheet 30 can be easily peeled off at the beginning of the use. The base sheet 30 covers the air introduction opening 14 in this manner in the packaged state before use. As a result, it is possible to prevent a user from mistakenly inserting a hand into the air introduction opening 14 and to prevent dusts or the like from entering into the inside of the sensitizing layer 2 via the air introduction opening 14. Although the base sheet 30 made of a paper is used in the present embodiment, the material forming the base sheet 30 is not limited to a particular material. It may be a film material or any other material.

As shown in FIG. 2(b), after the insertion of a hand through the insertion opening 5, the base sheet 30 is peeled off, thereby actually opening the air introduction opening 14. As a result, air is introduced between the first film 10 and the second film 12, thereby enabling the relative slide movement between the first film 10 and the second film 12. In other words, the glove 1 is now ready to be used.

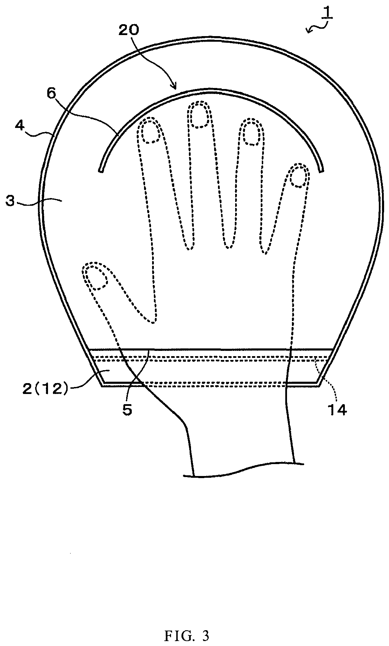

FIG. 3 is a plan view showing a state in which a hand is being inserted into the glove 1. As shown in this figure, the hand inserted between the sensitizing layer 2 and the external cover 3 is positioned at an optimum position for palpation without the fingertips reaching up to the peripheral edge of the sensitizing layer 2 since the tips, i.e., fingertips come into contact with the stopper 20. Specifically, the inserted hand is located at a position allowing the fingertips to smoothly slide the second film 12 relative to the first film 10 without being limited by the connected portion 4 connecting between the first film 10 and the second film 12.

In order to smoothly slide the second film 12 relative to the first film 10 without being influenced by the connected portion 4, the fingertips are preferably located on the slightly inner side of the peripheral edge of the glove 1 where the connected portion 4 is located. A distance from the peripheral edge of the glove 1 to the fingertips is not particularly limited and it can be suitably set depending on the intended use of the glove 1, or the like. For the purpose of the smooth movement of the fingertips, however, a distance from the connected portion 4 at the peripheral edge of the glove 1 to the fingertips for performing palpation (especially the index finger, the middle finger, and the fourth finger) is 10 mm or longer,
and preferably 15 mm or longer. In a breast cancer screening or the like, palpation is performed while moving fingertips broadly as if they drew a circle with a diameter in a range between 50 and 70 mm as will be described later. In such an intended use, the distance from the connected port ion 4 at the peripheral edge of the glove 1 to the fingertips for performing palpation is preferably in a range between 20 and 40 mm.

Although the stopper 20 is configured by the partially-connected portion 6 formed in an arc shape in the present embodiment, the stopper 20 may be configured by the partially-connected portion 6 having another shape such as a wave shape fitted to fingertips, for example. Alternatively, the stopper 20 may be configured by providing the partially-connected portions 6 separately for individual fingertips such as the index finger, the middle finger, and the fourth finger.

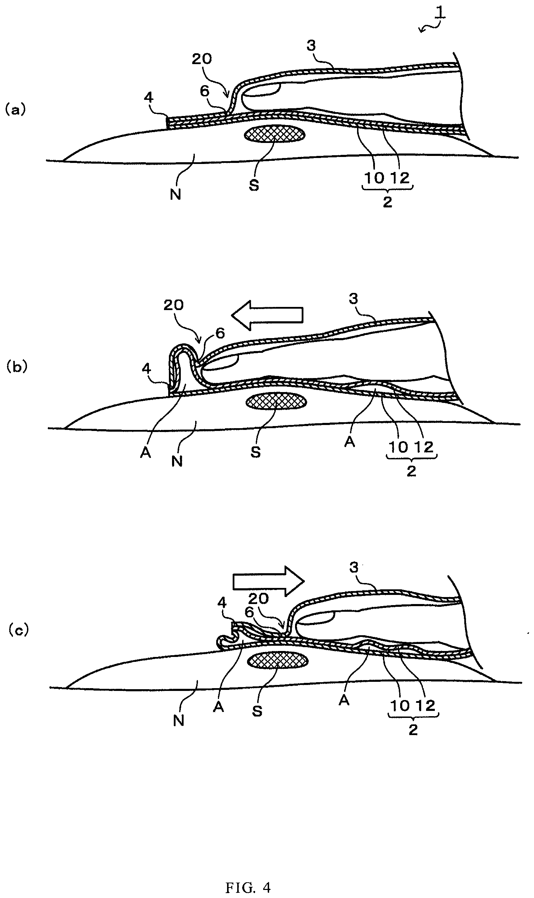

FIGs. 4(a) to 4(c) each are a cross-sectional view schematically showing a state during the use of the glove 1 in a breast cancer screening. In a breast cancer screening, as shown in FIG. 4(a), a hand is inserted between the sensitizing layer 2 and the external cover 3 first, and the sensitizing layer 2 is brought into contact with a breast N in the chest. Then, with the sensitizing layer 2 being pressed against the breast N, the breast N is touched by using the first joints in the balls of three fingers (the index finger, the middle finger, and the fourth finger), for example, while drawing a circle with a diameter of about 5 to 7 cm. Specifically, the screening is performed while pressing the breast N gently first and then pressing it a little harder. By repeating these several times, a detailed check is performed also for a region of the breast N with greater swelling.

When palpation is performed while drawing a circle of about 5 to 7 cm with a hand in this manner, fingertips are moved with the second film 12 being slid relative to the first film 10 as shown in FIGs. 4 (b) and 4 (c). Here, the first film 10 side is in close contact with the skin, resulting in a state integrated with the skin. The second film 12, on the other hand, is in close contact with a hand of a user, resulting in a state integrated with the fingertips. Since air A from outside is introduced between the first film 10 and the second film 12 through the air introduction opening 14, it never happens that the first film 10 and the second film 12 themselves are brought into adhering closely to each other due to a negative pressure or the like. Therefore, sliding resistance between the first film 10 and the second film 12 is reduced. Consequently, the interposition of the sensitizing layer 2 makes it possible to move the fingertips very smoothly along the skin. Thus, it is possible to sensitively detect minute contour on the surface of the breast N, a lump S inside the breast N, or the like.

Particularly in the present embodiment, fingertips are located by the stopper 20 on the inner side of the peripheral edge of the glove 1 where the connected portion 4 is located. As a result, when the fingertips are moved toward the peripheral edge of the glove 1, the second film 12 and the external cover 3 can appropriately bend between the fingertips and the connected portion 4 as shown in FIG. 4 (b). Moreover, when the fingertips are moved away from the peripheral edge of the glove 1, the first film 10 can appropriately bend between the fingertips and the connected portion 4 as shown in FIG. 4(c). As a result, it is possible to move the fingertips smoothly together with the second film 12 across a wide area without changing the positional relationship between the fingertips and the second film 12. Further, also in a case where the fingertips are moved toward the peripheral edge of the glove 1, an examination can be performed smoothly since it never happens that the fingertips come into contact with the connected portion 4 to push the whole glove 1 and the position of the first film 10 relative to the skin is thereby changed.

Next, modifications of the glove 1 will be described.

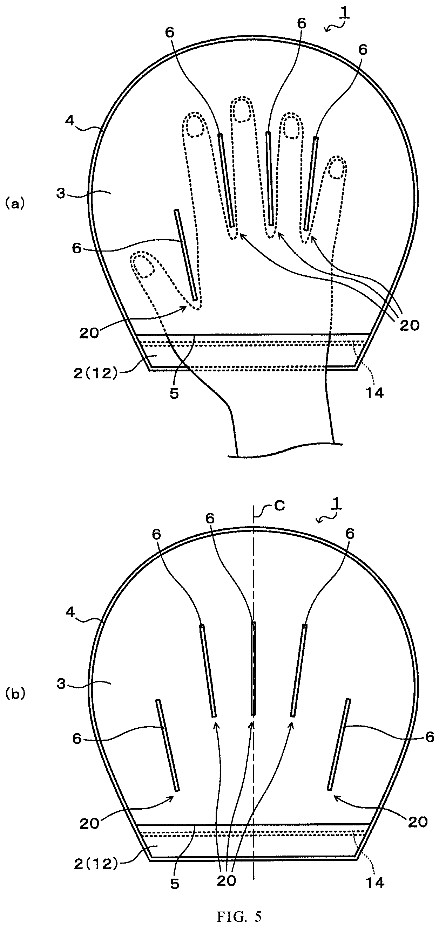

FIGs. 5 (a) and 5 (b) each are a plan view showing a not claimed comparative example in which the stoppers 20 are configured so as to come into contact with portions between fingers of a hand inserted between the sensitizing layer 2 and the external cover 3. In this example, the stoppers 20 are configured by forming the partially-connected portions 6 of a linear shape at positions between the fingers of the inserted hand. In this case, since the positions of the respective fingers can be defined more clearly, it is possible to sensitize touch perception more effectively depending on the intended use or the like. As described above, the stopper 20 may be any stopper as long as it comes into contact with any region of a hand to determine the positions of the fingertips.

In this case, the stoppers 20 may be provided so as to be fitted to a right hand (or a left hand) as shown in FIG. 5(a). Alternatively, the stoppers 20 may be provided symmetrically in a planar view as shown in FIG. 5(b) so that it is available for both of left and right hands. In other words, as shown in FIG. 5(b), the stoppers 20 (the partially-connected portions 6) may be provided line-symmetric with a center line C of the glove 1 so that it is available for both of left and right hands. Moreover, the partially-connected portion 6 forming the stopper 20 may be formed in another shape such as a dot shape, a circular shape, a dot-line shape, or the like. Moreover, the stoppers 20 may be provided between all of the fingers as shown in FIGs. 5(a) and 5(b) or only between some of the fingers.

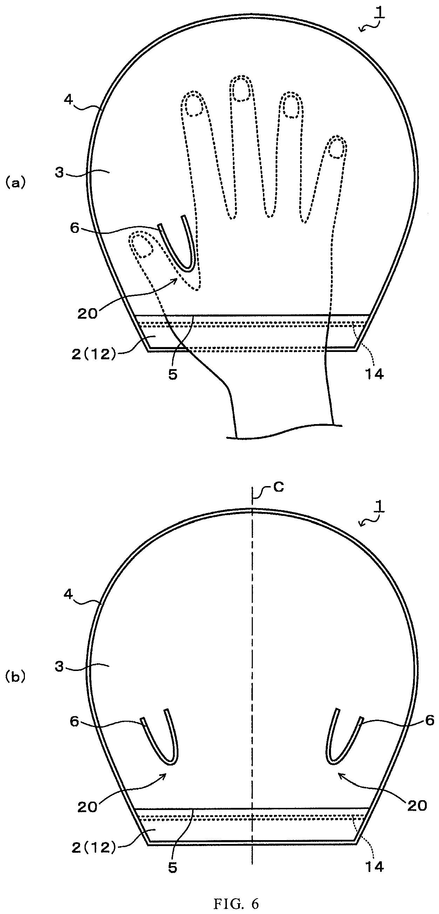

FIGs. 6(a) and 6(b) each are a plan view showing an example in which the stopper 20 is configured so as to come into contact with a portion between the thumb and the index finger. In this example, the stopper 20 is configured by forming the approximately U-shaped partially-connected portion 6 at a position between the thumb and the index finger of an inserted hand. In this case, it is possible to move the fingertips more smoothly since the partially-connected portion 6 connecting between the second film 12 and the external cover 3 does not exist in the vicinity of the fingertips performing palpation.

Also in this case, the stopper 20 may be provided at one location fitted to a right hand (or a left hand) as shown in FIG. 6 (a). Alternatively, the stoppers 20 may be provided at two locations symmetrically (line-symmetric with the center line C) in a planar view as shown in FIG. 6 (b) so that it is available for both of left and right hands.

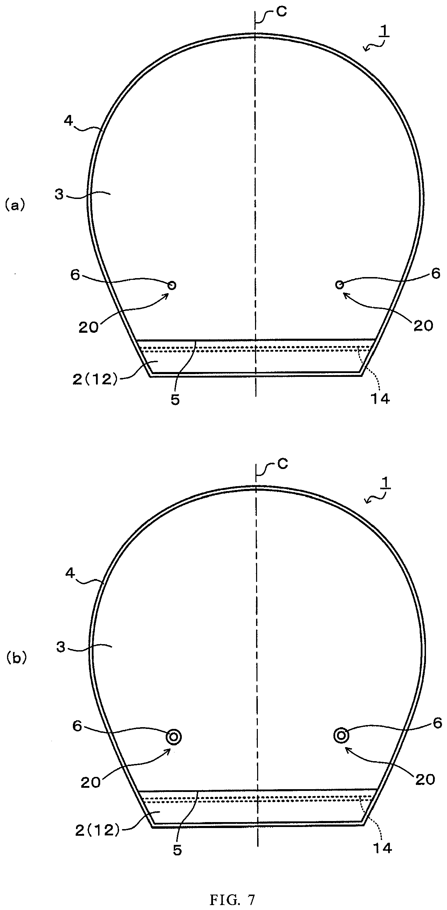

FIGs. 7 (a) and 7 (b) each are a plan view showing an example of another shape of the partially-connected portion 6 forming the stopper 20 to come into contact with a portion between the thumb and the index finger. The partially-connected portion 6 forming the stopper 20 in this case may be formed in a dot shape, for example, as shown in FIG. 7 (a). Alternatively, it may be formed in a circular shape as shown in FIG. 7 (b). Although illustration in a figure is omitted, it may be formed in another shape such as an elliptical shape, a V-shape, or the like. Further, in order to reliably place the stopper 20 between the thumb and the index finger, an indication showing the suitable position of the thumb may be provided.

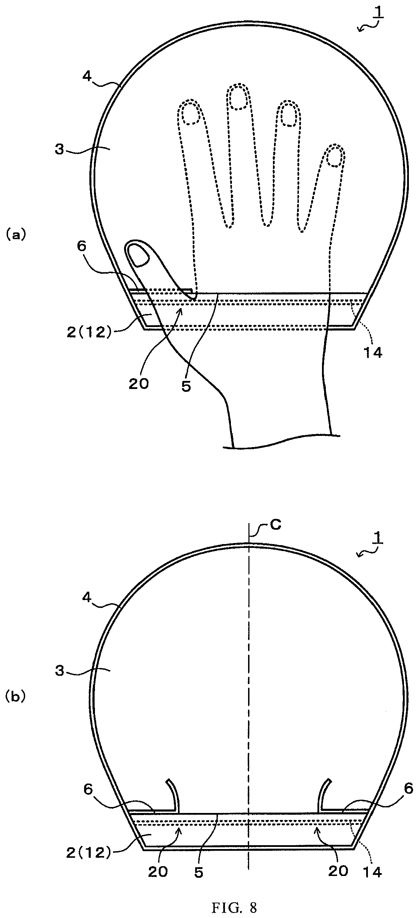

FIGs. 8 (a) and 8 (b) each are a plan view showing an example of the stopper 20 configured by closing part of the insertion opening 5. In this example, part of the insertion opening 5 is closed by providing the partially-connected portion 6 at a left end portion (or a right end portion) of the insertion opening 5 in a planar view. As a result, it becomes impossible to insert the thumb between the sensitizing layer 2 and the external cover 3, and the position of the fingertips is thereby suitably located. In other words, the stopper 20 that comes into contact with a portion between the thumb and the index finger is configured by the partially-connected portion 6 closing part of the insertion opening 5 in this example.

Since no partially-connected portion 6 exists inside the external cover 3 in this case, fingertips can be moved more smoothly. Moreover, an inserted portion of a hand inserted between the second film 12 and the external cover 3 is reduced in this case. Thus, it is possible to configure the glove 1 compactly so as to be fitted to the inserted portion of a hand, thereby achieving reductions in the manufacturing cost and distribution cost thereof and simplifying the handling thereof.

Also in this case, the stopper 20 may be provided at one location fitted to a right hand (or a left hand) as shown in FIG. 8 (a). Alternatively, the stoppers 20 may be provided at two locations symmetrically (line-symmetric with the center line C) in a planar view as shown in FIG. 8 (b) so that it is available for both of left and right hands. Moreover, the partially-connected portion 6 forming the stopper 20 may be formed in a shape extending toward the inner side from the insertion opening 5 as shown in FIG. 8 (b).

Further, the partially-connected portion 6 forming the stopper 20 in this case is not limited to the partially-connected portion connecting only between the second film 12 and the external cover 3. It may be a partially-connected portion connecting all of the first film 10, the second film 12, and the external cover 3 together. By employing the partially-connected portion 6 connecting all of the first film 10, the second film 12, and the external cover 3 together, the connected portion 4 and the partially-connected portion 6 can be formed in one step. As a result, it is possible to reduce the manufacturing cost thereof. Moreover, an indication expressing that the thumb is placed outside may be provided also in this case.

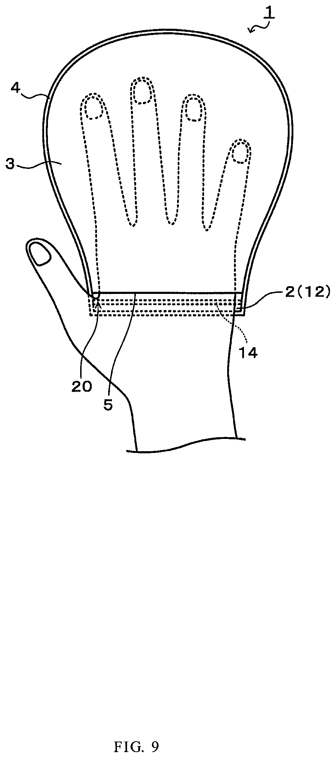

FIG. 9 is a plan view showing an example in which the insertion opening 5 is formed to have a width allowing a hand to be inserted partially. In this example, by narrowing a width of the glove 1 on the side of the insertion opening 5, the width of the insertion opening 5 is set to be a width into which only a portion from the index finger to the little finger above the base of the thumb can be inserted. Therefore, the stopper 20 to come into contact with a portion between the thumb and the index finger is configured by part of the peripheral edge of the insertion opening 5 in this example.

In this case, since there is no need to provide the partially-connected portion 6, the fingertips can be moved more smoothly and the manufacturing cost thereof can be reduced. Further, in this case, the glove 1 can be configured in an extremely compact manner so as to be fitted to the inserted portion of a hand as shown in FIG. 9. As a result, itispossible to reduce the manufacturing cost and distribution cost thereof and to simplify the handling of the glove 1. In this case, it is also possible to form the insertion opening 5 so as to have a width into which only the index finger, the middle finger, and the fourth finger can be inserted, a width into which only the index finger and the middle finger can be inserted, or the like, for example.

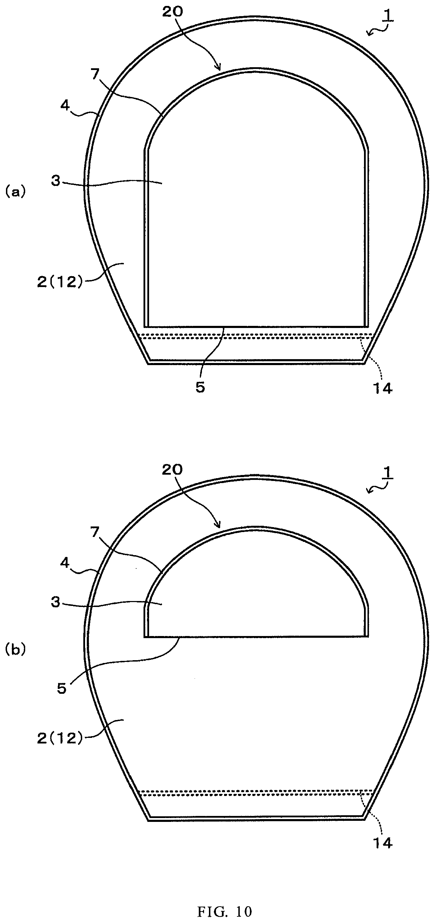

FIGs. 10 (a) and 10 (b) each are a plan view showing a configuration example in which the size of the external cover 3 in a planar view is made smaller than that of the sensitizing layer 2. In this example, by making the size of the external cover 3 in a planar view smaller than that of the sensitizing layer 2, an external cover connected portion 7 connecting between the external cover 3 and the second film 12 at the peripheral edge of the external cover 3 is located on the inner side than the peripheral edge of the sensitizing layer 2 in a planar view. Therefore, the external cover connected portion 7 forms the stopper 20 in this example. In this case, by suitably setting the size and shape and the position of the external cover 3, the position of a hand can be limited more definitely. Moreover, since it can be easily determined in this case which side is the sensitizing layer 2 to be in contact with an object, it is possible to prevent an improper use such as the use with the external cover 3 thereof being in contact with an object.

In this case, the external cover 3 may be configured to have a size enabling the insertion of approximately the entire hand as shown in FIG. 10(a). Alternatively, the external cover 3 may be configured to have a size enabling the insertion of the fingertips only as shown in FIG. 10 (b), for example. Alternatively, the external cover 3 may be configured to have a size enabling the insertion of only a portion from the index finger to the little finger above the base of the thumb in the similar manner to the example shown in FIG. 8, a size enabling the insertion of the index finger, the middle finger, and the fourth finger only, or the like, for example. Moreover, the shape of the external cover 3 may be another shape such as a shape fitted to the shape of a hand or fingers, for example.

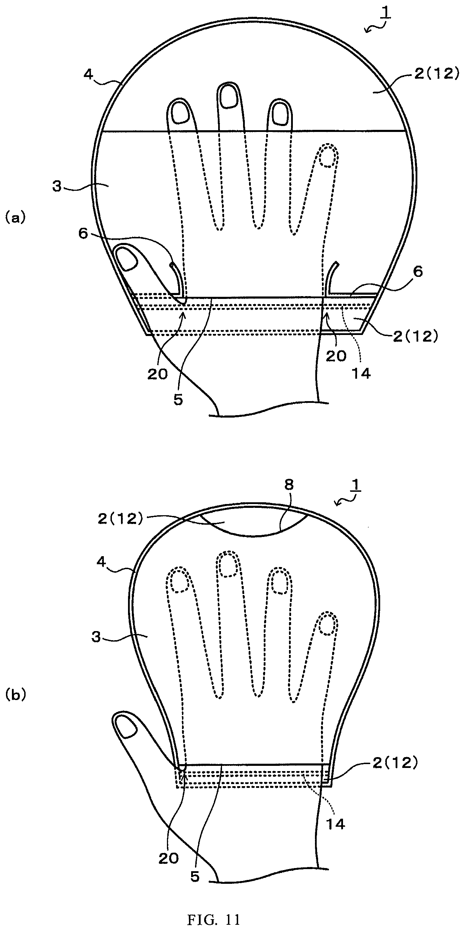

FIGs. 11 (a) and 11 (b) each are a view showing an example in which the tip side (the fingertip side) of the external cover 3 is opened. In the example shown in FIG. 11 (a), the external cover 3 is configured to have an approximately strip shape, and the tip side of the external cover 3 is made opened by not connecting the external cover 3 on the tip side (fingertip side) of a hand inserted between the sensitizing layer 2 and the external cover 3 with the second film 12. Alternatively, in the example shown in FIG. 11 (b), a cutout portion 8 of an approximately arc shape is provided on the tip side of the external cover 3, and the tip side of the external cover 3 is opened by not connecting the cutout portion 8 with the second film 12.

By configuring the external cover 3 not in a bag shape but to open the tip side thereof at least partially as described above, fingertips performing palpation can be moved more smoothly and a ventilation characteristic thereof can be improved, thereby obtaining a pleasant usability. Further, since distinguishing between the sensitizing layer 2 and the external cover 3 can be made easier in this case, it is possible to prevent an improper use such as the use with the external cover 3 being in contact with an object.

In this case, the fingertips of a hand inserted between the sensitizing layer 2 and the external cover 3 maybe exposed as shown in FIG. 11 (a). Alternatively, it may be configured so that no fingertips are exposed as shown in FIG. 11(b). Moreover, the shape of the cutout portion 8 is not limited to a particular shape. An appropriate shape such as a trapezoidal shape or a linear shape may be employed. Further, a plurality of the cutout portions 8 may be provided. Needless to say, the tip side of the external cover 3 may be opened in the other embodiments shown in FIGs. 1, 5, 6, 7, and 10.

As described above, the glove 1 according to the present embodiment is a glove for sensitizing touch perception, including: the sensitizing layer 2 to be in contact with an object; and the external cover 3 for fixing a hand to the sensitizing layer 2, for use in detecting minute irregularities of the object via the sensitizing layer 2 by means of a hand inserted between the sensitizing layer 2 and the external cover 3. The sensitizing layer 2 includes: the first film 10 to be in contact with the object; the second film 12 layered on the first film 10; and the air introduction opening 14 for introducing air between the first film 10 and the second film 12 to allow the second film 12 to slide relative to the first film 10. The external cover 3 or the sensitizing layer 2 includes the stopper 20 for positioning the tip of the hand inserted between the sensitizing layer 2 and the external cover 3 on the inner side of the peripheral edge of the sensitizing layer 2.

By providing the stopper 20 in this manner, fingertips can be placed at a position where the relative movement between the first film 10 and the second film 12 is not disturbed by the connected portion 4 provided at the peripheral edge of the sensitizing layer 2. As a result, it becomes possible to move the fingertips smoothly across a wide area without influencing subtle touch perception, thereby enabling a highly-accurate and smooth examination by means of palpation . In general, when a hand is inserted into an object of a glove shape, a person habitually seeks to insert one's hand deep inside securely. However, this is prevented by the placement of the stopper 20 and the fingertips performing palpation can be thereby located at a position enabling an adequate examination.

Moreover, the first film 10 and the second film 12 are directly slid to each other without using a lubricating liquid therein in the present embodiment. As a result, there is no need to worry about liquid spill or the like, and it is possible to achieve reductions in size and weight thereof. For example, according to the present embodiment, the glove 1 in a state being attached to the base sheet 30 (see FIG. 2 (a)) is folded up compactly and it can be distributed to the market in a packaged state with a size of about 5 centimeters square and about two millimeters thick and a weight of 10 grams or less.

Moreover, by sealing the air introduction opening 14 with the base sheet 30, it is possible to prevent a hand from being inserted mistakenly into the air introduction opening 14 and to prevent dusts from entering into the inside of the sensitizing layer 2 to influence the judgment of palpation. As a result, it is possible to obtain a product user-friendly in all aspects from the distribution process to the in-use process.

Moreover, according to the present embodiment, the manufacturing cost thereof can be reduced dramatically to an inexpensive price as compared to the conventional technique . Thus, it is possible to use the glove 1 as a disposable glove. In this case, since the glove 1 can be replaced with another for each examination, an examination can be performed while keeping a high level of hygiene. Moreover, a low-pollution polymer material is used as the material in the present embodiment. Thus, an influence on the environment can be minimized even in a case where the glove 1 is used as a disposable glove.

Note that the shape of the glove 1 is not limited to the shape shown in the present embodiment. For example, it may be another shape such as a rectangular shape or a pentagon shape. Further, the glove 1 may be conf igured in a glove shape into which respective fingers can be inserted independently or in a mitten shape into which only the thumb can be inserted independently.

Moreover, although the present embodiment shows the example in which the first film 10, the second film 12, and the external cover 3 are formed by a synthetic resin, the present invention is not limited thereto. For example, other materials such as various fabrics or non-woven fabrics may be used. Further, part of the first film 10, the second film 12, and the external cover 3 may be formed by different materials, e.g., only the external cover 3 is formed by a non-woven fabric, etc.

Moreover, although the present embodiment shows the example in which the sensitizing layer 2 is formed by the two films, i.e., the first film 10 and the second film 12, the present invention is not limited thereto. The sensitizing layer may be formed by layering three or more films.

Moreover, although the present embodiment shows the example in which no lubricant or the like is used between the first film 10 and the second film 12 in the sensitizing layer2, the present invention is not limited thereto. Various liquid lubricants or solid lubricants, various powders, or the like may be inserted between the films depending on the materials of the films, the intended use of the glove 1, and the like.

Moreover, the shape of the air introduction opening 14 is not limited to the shape shown in the present embodiment. It may be another shape such as the formation of the air introduction opening 14 by means of a plurality of circular holes, for example.

The method for connecting together the first film 10, the second film 12, and the external cover 3 in the connected portion 4, the partially-connected portion 6, and the external cover connected portion 7 is not limited to a particular method. An appropriate known method according to the characteristics of the material, the manufacturing process, and the like can be employed such as fusion, welding, adhesion by means of an adhesive agent, or sewing.

Moreover, although the present embodiment shows the example in which the stopper 20 is formed by connecting the second film 12 and the external cover 3 together, the present invention is not limited thereto. For example, the stopper 20 may be formed by another known method, e.g., the stopper 20 is formed by placing between the second film 12 and the external cover 3 another member capable of coming into contact with any region of a hand.

Moreover, the shape of the external cover 3 is not limited to the shape shown in the present embodiment. Various shapes may be employed, e.g., forming the insertion opening 5 in an arc shape, providing the cutout portion 8 of a varied shape at an appropriate position, and the like. Further, an indication expressing that it is not a surface to be in contact with an object may be shown on the surface of the external cover 3 by means of printing or the like.

Although the present embodiment shows the usage for the medical and treatment purposes mainly such as a breast cancer screening, the present invention is not limited thereto. It can be used in any situation where touch perception needs to be sensitized. It can be used in a different intended use, e.g., when a surface finishing state of an industrial product is checked finally, when surface irregularities of an art object are checked, or the like.

Although the embodiments of the present invention are described above, the glove for sensitizing touch perception according to the present invention is not limited to the embodiments described above. It is to be understood that various changes may be made without departing from the scope of the present invention.

### Industrial Applicability

The glove for sensitizing touch perception according to the present invention can be used in the field of surface examination for various goods such as industrial products or art objects in addition to the medical field such as a breast cancer screening.

### Reference Signs List

- 1: glove for sensitizing touch perception
- 2: sensitizing layer
- 3: external cover
- 4: connected portion
- 5: insertion opening
- 6: partially-connected portion
- 7: external cover connected portion
- 10: first film
- 12: second film
- 14: air introduction opening
- 20: stopper

## Claims

1. A glove (1) for sensitizing touch perception, comprising: a sensitizing layer (2) to be in contact with an object; and an external cover (3) for fixing a hand to the sensitizing layer (2), for use in detecting minute irregularities of the object via the sensitizing layer (2) by means of a hand inserted between the sensitizing layer (2) and the external cover (3), wherein
the sensitizing layer (2) includes:
a first film (10) to be in contact with the object;
a second film (12) layered on the first film (10); and
an air introduction opening (14) for introducing air between the first film (10) and the second film (12) to allow the second film (12) to slide relative to the first film (10),
**characterized in that**
the external cover (3) or the sensitizing layer (2) includes a stopper (20) for positioning a fingertip of an index finger of a hand inserted between the sensitizing layer (2) and the external cover (3) on an inner side of a peripheral edge of the sensitizing layer (2) by 10 mm or more,
the stopper (20) is formed so as to come into contact with a fingertip of a hand, and
the stopper (20) is formed by connecting the sensitizing layer (2) and the external cover (3) together or at least at part thereof.

2. The glove (1) for sensitizing touch perception according to claim 1, wherein: the external cover (3) is configured in a size smaller than that of the sensitizing layer (2); at least part of a peripheral edge of the external cover (3) is located on an inner side as compared to the peripheral edge of the sensitizing layer (2); and the peripheral edge of the external cover (3) located on the inner side as compared to the peripheral edge of the sensitizing layer (2) serves as the stopper (20).

3. The glove (1) for sensitizing touch perception according to claim 1 or 2, wherein the stoppers (20) are symmetrically provided so that the glove (1) is available for both of left and right hands.

4. The glove (1) for sensitizing touch perception according to any of claims 1 to 3, wherein the stopper (20) is formed so that a fingertip of a hand inserted between the sensitizing layer (2) and the external cover (3) is located on the inner side from the peripheral edge of the sensitizing layer (2) by 20 to 40 mm.

5. The glove (1) for sensitizing touch perception according to any of claims 1 to 4, wherein at least part of the external cover (3) is opened on a tip side of a hand inserted between the sensitizing layer (2) and the external cover (3).

## Patentansprüche

1. Handschuh (1) zum Sensibilisieren einer Tastempfindung, mit: einer Sensibilisierungslage (2), die mit einem Gegenstand in Kontakt sein soll; und einer Außenabdeckung (3) zum Fixieren einer Hand an die Sensibilisierungslage (2), zur Verwendung beim Auffinden von geringfügigen Unregelmäßigkeiten des Gegenstandes über die Sensibilisierungslage (2) mittels einer zwischen der Sensibilisierungslage (2) und der Außenabdeckung (3) eingesetzten Hand, wobei
die Sensibilisierungslage (2) Folgendes umfasst:
eine erste Schicht (10), die mit dem Gegenstand in Kontakt sein soll;
eine zweite Schicht (12), die an der ersten Schicht (10) schichtweise angeordnet ist; und
eine Lufteinleitungsöffnung (14) zum Einleiten von Luft zwischen die erste Schicht (10) und die zweite Schicht (12), um ein Gleiten der zweiten Schicht (12) bezüglich der ersten Schicht (10) zu ermöglichen,
**dadurch gekennzeichnet, dass**
die Außenabdeckung (3) oder die Sensibilisierungslage (2) einen Stopper (20) zum Positionieren einer Fingerspitze eines Zeigefingers einer zwischen der Sensibilisierungslage (2) und der Außenabdeckung (3) eingesetzten Hand an einer inneren Seite eines Umfangsrands der Sensibilisierungslage (2) um 10 mm oder mehr umfasst,
der Stopper (20) ausgebildet ist, um mit einer Fingerspitze einer Hand in Kontakt zu kommen, und
der Stopper (20) durch ein Verbinden der Sensibilisierungslage (2) mit der Außenabdeckung (3) oder mindestens einem Teil davon ausgebildet ist.

2. Handschuh (1) zum Sensibilisieren einer Tastempfindung gemäß Anspruch 1, wobei: die Außenabdeckung (3) in einer Größe gestaltet ist, die kleiner als die der Sensibilisierungslage (2) ist; mindestens ein Teil eines Umfangsrands der Außenabdeckung (3) an einer inneren Seite im Vergleich mit dem Umfangsrand der Sensibilisierungslage (2) gelegen ist; und der Umfangsrand der Außenabdeckung (3), der an der inneren Seite im Vergleich mit dem Umfangsrand der Sensibilisierungslage (2) gelegen ist, als der Stopper (20) dient.

3. Handschuh (1) zum Sensibilisieren einer Tastempfindung gemäß Anspruch 1 oder 2, wobei die Stopper (20) symmetrisch vorgesehen sind, sodass der Handschuh (1) sowohl für eine linke als auch eine rechte Hand zur Verfügung steht.

4. Handschuh (1) zum Sensibilisieren einer Tastempfindung gemäß einem der Ansprüche 1 bis 3, wobei der Stopper (20) ausgebildet ist, sodass eine Fingerspitze einer zwischen der Sensibilisierungslage (2) und der Außenabdeckung (3) eingesetzten Hand an der inneren Seite von dem Umfangsrand der Sensibilisierungslage (2) um 20 bis 40 mm gelegen ist.

5. Handschuh (1) zum Sensibilisieren einer Tastempfindung gemäß einem der Ansprüche 1 bis 4, wobei mindestens ein Teil der Außenabdeckung (3) an einer vordersten Seite einer zwischen der Sensibilisierungslage (2) und der Außenabdeckung (3) eingesetzten Hand geöffnet ist.

## Revendications

1. Gant (1) pour sensibiliser à la perception tactile comprenant : une couche de sensibilisation (2) destinée à être en contact avec un objet ; et une couverture externe (3) pour fixer une main sur la couche de sensibilisation (2), destinée à être utilisée pour détecter de minuscules irrégularités de l'objet via la couche de sensibilisation (2) au moyen d'une main insérée entre la couche de sensibilisation (2) et la couverture externe (3), dans lequel :
la couche de sensibilisation (2) comprend :
un premier film (10) destiné à être en contact avec l'objet ;
un second film (12) disposé en couche sur le premier film (10) ; et
une ouverture d'introduction d'air (14) pour introduire l'air entre le premier film (10) et le second film (12) pour permettre au second film (12) de glisser par rapport au premier film (10),
**caractérisé en ce que** :
la couverture externe (3) ou la couche de sensibilisation (2) comprend une butée (20) pour positionner le bout d'un index de la main insérée entre la couche de sensibilisation (2) et la couverture externe (3) sur un côté interne d'un bord périphérique de la couche de sensibilisation (2) sur 10 mm ou plus,
la butée (20) est formée afin de venir en contact avec le bout d'un doigt d'une main, et
la butée (20) est formée en raccordant la couche de sensibilisation (2) et la couverture externe (3) ensemble ou au moins une partie de cette dernière.

2. Gant (1) pour sensibiliser à la perception tactile selon la revendication 1, dans lequel : la couverture externe (3) est configurée dans une taille inférieure à celle de la couche de sensibilisation (2) ; au moins une partie d'un bord périphérique de la couverture externe (3) est positionnée sur un côté interne par rapport au bord périphérique de la couche de sensibilisation (2) ; et le bord périphérique de la couverture externe (3) positionné sur le côté interne par rapport au bord périphérique de la couche de sensibilisation (2) sert de butée (20) .

3. Gant (1) pour sensibiliser à la perception tactile selon la revendication 1 ou 2, dans lequel les butées (20) sont prévues de manière symétrique de sorte que le gant (1) est disponible pour les mains droite et gauche.

4. Gant (1) pour sensibiliser à la perception tactile selon l'une quelconque des revendications 1 à 3, dans lequel la butée (20) est formée de sorte que le bout d'un doigt d'une main insérée à l'intérieur de la couche de sensibilisation (2) et la couverture externe (3) est positionné sur le côté interne à partir du bord périphérique de la couche de sensibilisation (2) sur 20 à 40 mm.

5. Gant (1) pour sensibiliser à la perception tactile selon l'une quelconque des revendications 1 à 4, dans lequel au moins une partie de la couverture externe (3) est ouverte sur un côté de bout d'une main insérée entre la couche de sensibilisation (2) et la couverture externe (3).
